# FASCICULE DE BREVET EUROPEEN

(11) **EP 1 017 832 B1**
(45) Date de publication et mention de la délivrance du brevet: **15.12.2004**
(21) Numéro de dépôt: 98945366.7
(22) Date de dépôt: 23.09.1998
(51) Int. Cl.: C12N 15/82, C12N 15/29, A01H 1/02, A01H 5/00, A01H 5/10

(54) **PROMOTEUR SPECIFIQUE DES MICROSPORES ET PROCEDE D'OBTENTION DE PLANTES HYBRIDES**
MIKROSPORE-SPEZIFISCHER PROMOTOR UND EINE VERFAHREN ZUR HERSTELLUNG VON HYBRIDPFLANZEN
MICROSPORE-SPECIFIC PROMOTER AND METHOD FOR OBTAINING HYBRID PLANTS

(30) Priorité: 23.09.1997 FR 9711812
(43) Date de publication de la demande: 12.07.2000
(73) Titulaire: INSTITUT NATIONAL DE LA RECHERCHE AGRONOMIQUE (INRA), 75007 Paris Cédex 07 (FR)
(72) Inventeur: DROUAUD, Jan, F-78000 Versailles (FR); FOURGOUX, Agnès, F-78330 Fontenay Le Fleury (FR); PELLETIER, Georges, F-91440 Bures sur Yvette (FR); GUERCHE, Philippe, F-92170 Vanves (FR)
(74) Mandataire: Ahner, Francis
(86) Numéro de dépôt international: PCT/FR1998/002042
(87) Numéro de publication internationale: WO 1999/015678

(56) Documents cités:
- EP-A- 0 329 308
- EP-A- 0 436 467
- EP-A- 0 790 311
- WO-A-90/08828
- WO-A-90/08830
- WO-A-92/18625
- WO-A-94/13809
- WO-A-94/21804
- WO-A-94/25613
- WO-A-96/16182
- SUZUKI, G., ET AL.: "Brassica rapa DNA for S-locus glycoprotein, complete cds." EMBL SEQUENCE DATABASE ACCESSION NO. D88192,10 juillet 1997, XP002068717
- SUZUKI, G., ET AL.: "Brassica rapa DNA for S-receptor kinase, complete cds." EMBL SEQUENCE DATABASE ACCESSION NO. D88193,10 juillet 1997, XP002068718
- NAKAMURA, Y., ET AL.: "Arabidopsis thaliana genomic DNA, chromosome 5, P1 clone: MKP11" EMBL SEQUENCE DATABASE ACCESSION NO. AB005238, XP002093097
- KANDASAMY, M.K., ET AL.: "Ablation of papillar cell function in Brassica flowers results in the loss of stigma receptivity to pollination" THE PLANT CELL, vol. 5, 1993, pages 263-275, XP002067958
- DENIS,M., ET AL.: "Expression of engineered nuclear male sterility in Brassica napus" PLANT PHYSIOLOGY, vol. 101, 1993, pages 1295-1304, XP002009916

## Description

La présente invention concerne notamment un promoteur spécifique des microspores et un procédé d'obtention de plantes hybrides.

La microspore correspond à un stade précis du développement du gamète mâle chez les plantes supérieures. La gamétogenèse mâle a lieu dans un organe spécialisé, l'anthère, et comprend *sensu stricto* la différenciation de cellules diploïdes en grains de pollen haploïdes. Chaque cellule diploïde appelée cellule sporogène subit une méïose pour produire quatre microspores haploïdes qui se différencient par la suite pour donner des grains de pollen matures.

Connaître et savoir manipuler les facteurs moléculaires qui contrôlent le développement de la microspore est un enjeu considérable non seulement d'un point de vue de la recherche fondamentale mais aussi d'un point de vue de l'amélioration des plantes. En effet, cette connaissance permet de maîtriser la production de grains de pollen et par conséquent la reproduction de la plante.

Une telle maîtrise passe par l'obtention de plantes totalement stériles pour l'un de leur gamète de façon à empêcher l'autofécondation.

Jusqu'à présent, la stérilité mâle des plantes, moins complexe que la stérilité femelle, a été largement étudiée mais nécessite l'utilisation de systèmes génétiques relativement lourds à mettre en oeuvre pour la production commerciale de semences hybrides. Un type de stérilité mâle très utilisée est la stérilité mâle cytoplasmique qui consiste en l'obtention :
- d'une lignée femelle dont le caractère mâle-stérile est transmis par le cytoplasme; on appelle un tel cytoplasme un "cytoplasme inducteur de stérilité mâle" ; ces "cytoplasmes inducteurs" pour une espèce donnée sont en général soit découverts dans la nature, soit observés parfois chez des plantes résultant de croisements interspécifiques (fécondation croisée, fusion de protoplastes etc...),
- d'une lignée "mainteneuse" de stérilité dont le cytoplasme est normal, et
- d'une lignée restauratrice de fertilité si l'on récolte les graines et/ou les fruits de la plante hybride.

Dans la lignée femelle (porteuse du cytoplasme inducteur de stérilité) tous les grains de pollen sont tués. Il est donc nécessaire pour multiplier et améliorer cette lignée de disposer d'une lignée ne portant ni le cytoplasme inducteur (donc produisant des grains de pollen) ni le gène de restauration. Cette lignée est dite "mainteneuse" de stérilité car le croisement avec la lignée femelle donne une descendance entièrement femelle.

La restauration de la fertilité est réalisée dans l'hybride par le croisement du parent femelle (portant le cytoplasme mâle stérile) avec le parent comprenant un gène nucléaire de la restauration (la lignée restauratrice), ce croisement permettant l'obtention de plantes hybrides fertiles qui produiront des graines par autofécondation.

Dans le cas de la stérilité nucléaire sporophytique, il a été décrit, par exemple, des systèmes permettant de tuer les cellules mères des microspores au moyen d'une RNAse et par conséquent d'obtenir des plantes dépourvues de gamètes mâles. La fertilité est restaurée au moment du croisement de la lignée ne produisant plus de gamètes mâles avec une autre lignée portant un inhibiteur de la RNAse, les graines résultant de ce croisement comprenant à la fois le gène cytotoxique et son inhibiteur.

La présente invention propose quant à elle d'obtenir des plantes présentant une stérilité mâle gamétophytique et incapables de produire des grains de pollen. Elle consiste à mettre en oeuvre une région promotrice contrôlant l'expression spécifiquement dans les microspores d'un gène codant pour une molécule cytotoxique tout en disposant d'un moyen permettant l'inhibition contrôlée de cette toxicité afin d'obtenir une lignée de plantes génitrices homozygotes totalement stériles quant à leurs gamètes mâles et ensuite obtenir des plantes hybrides fertiles (produisant un grain de pollen viable sur deux), donc capables de produire des graines, sans avoir recours à l'utilisation d'un gène de restauration de la fertilité.

Jusqu'à présent, un seul gène s'exprimant spécifiquement dans la microspore a été décrit chez le tabac (Oldenholf et al., 1996). Ce gène ne possède pas d'homologue chez les Brassicacées comme cela résulte d'une expérience de Southern blot sur de l'ADN génomique de *Brassica oleracea* (données non montrées).

La présente invention a donc pour objet une séquence nucléotidique dont il a été démontré que le gène correspondant s'exprime spécifiquement dans la microspore, cette séquence nucléotidique correspond à SEQ ID N° 3.

Par conséquent, la présente invention a pour objet une séquence nucléotidique correspondant à tout ou partie
de la séquence selon SEQ ID N° 3.

Dans le cadre de la présente invention, la partie la plus intéressante de cette séquence nucléotidique est la région promotrice définie comme étant la séquence précédant (côté 5') le codon de début de traduction (ATG). Cependant, au stade actuel des connaissances de la séquence nucléotidique selon SEQ ID N° 3, trois ATG ont été mis en évidence. Un en position 1965, un autre en position 2085 et un troisième en position 2112. Il semblerait que l'ATG fonctionnel soit celui situé en position 2085. Ceci n'est cependant pas confirmé, c'est la raison pour laquelle la plus grande région promotrice envisageable concernant SEQ LD N° 3 s'étend du nucléotide 1 au nucléotide 2111 et préférentiellement du nucléotide 1 au nucléotide 2084.

Cette région promotrice précède donc à l'état naturel, une séquence codante (orf) qui est exprimée spécifiquement dans les microspores et dans le cas où cet orf est remplacé (par manipulation génétique) par un autre orf dont le produit est une molécule cytotoxique, cette dernière est susceptible de ne détruire que lesdites microspores.

La présente invention a donc également pour objet des vecteurs d'expression cellulaire comprenant une séquence promotrice telle que ci-dessus décrite placée en amont d'une séquence d'ADN codant pour un produit cytotoxique.

Avantageusement, le produit cytotoxique en question est une protéase. En effet, lorsque la protéase s'exprimera spécifiquement dans les microspores, elle en détruira toutes les protéines, ce à quoi la microspore ne pourra pas survivre. Préférentiellement, la protéase est une subtilisine et en particulier la subtilisine BPN' de *Bacillus amyloliquefasciens.* Cette subtilisine BPN' fait partie de la famille des subtilisines que l'on trouve chez de nombreux organismes et qui sont des protéases connues pour couper les protéines au niveau des sérines.

Il s'agit donc d'introduire un vecteur conforme à l'invention dans une souche bactérienne susceptible de réaliser la transformation de cellules de plantes telle qu'*Agrobacterium tumefaciens*. Ceci peut notamment être réalisé par la méthode d'infiltration de plantes *d'Arabidopsis thaliana* décrite par Bechtold et al., 1993. Cette technique consiste à introduire la bactérie dans les cellules des hampes florales par infiltration sous vide. Les plantes sont ensuite repiquées en serre et leur graines récoltées. Environ une graine sur mille donnent naissance à des plantes dont toutes les cellules portent le transgène. La transformation d'autres plantes, et notamment du colza, peut être réalisée par l'intermédiaire d'*Agrobacterium tumefaciens* et/ou *Agrobacterium rhizogenes* à l'aide de diverses techniques, maintenant classiques (transformation de disques foliaires, d'hypocotyles, de hampes florales etc...) associant une phase de coculture de la bactérie avec les tissus végétaux, suivie de la sélection et de la régénération des cellules transformées en plantes entières. D'autres techniques de transformation ne font pas intervenir cette bactérie et permettent de transférer directement le gène cloné dans des cellules ou des tissus (electroporation, canon à particules etc...) et de sélectionner et d'obtenir des plantes transformées (revue par Siemens and Schieder).

La présente invention a également pour objet les cellules de plantes transformées par un vecteur conforme à l'invention ainsi que des plantes comprenant lesdites cellules.

L'invention a également pour objet des plantes présentant une stérilité mâle gamétophytique à fertilité inductible comprenant un gène codant pour un produit cytotoxique spécifique des gamètes mâles.

Comme indiqué précédemment, la présente invention permet donc l'obtention de plantes présentant une stérilité mâle gamétophytique inhibant toute production de grains de pollen. Cependant, ces plantes homozygotes quant à leur stérilité mâle ne peuvent être obtenues qu'après autofécondation de plantes préalablement transformées par un vecteur conforme à l'invention, c'est-à-dire hémizygotes quant à leur stérilité mâle et chez lesquelles on a restauré provisoirement la fertilité des grains de pollen porteurs de la stérilité gamétophytique pour leur permettre de réaliser une autofécondation.

Un moyen d'obtenir des plantes homozygotes pour ce gène serait d'utiliser la gynogenèse, technique qui consiste à régénérer des plantes haploïdes doublées à partir de culture d'ovules ou d'ovaires. Il s'agit dans ce cas d'obtenir la formation d'une plante homozygote diploïde à partir du gamète femelle haploïde. La gynogenèse est applicable à un certain nombre d'espèces végétales mais il n'est pas envisageable de produire un grand nombre de plantes homozygotes pour le transgène en question par cette technique, car sa mise en oeuvre est délicate et son efficacité reste le plus souvent très faible.

La présente invention concerne également un procédé d'obtention de plantes présentant une stérilité mâle gamétophytique à fertilité inductible comprenant :
- l'insertion dans des plantes de lignée A d'un gène dont le produit d'expression est cytotoxique pour les microspores, et
- l'obtention des plantes ne produisant pas de gamètes mâles.

Plus particulièrement, le procédé d'obtention de plantes présentant une stérilité mâle gamétophytique à fertilité inductible conforme à l'invention comprend les étapes de :
a) transformation de plantes d'une lignée A avec un vecteur conforme à l'invention,
b) induction de la fertilité des plantes obtenues en a) par inhibition de la cytotoxicité du produit,
c) autofécondation des plantes fertiles obtenues en b),
d) sélection des plantes ne produisant pas de gamètes mâles issues de c),
e) multiplication des plantes obtenues en d) par reproduction des étapes b) et c).

Ainsi, à l'étape a) du procédé ci-dessus, on transforme une lignée A avec un vecteur conforme à l'invention c'est-à-dire comprenant une séquence promotrice spécifique des microspores placée en amont d'un gène codant pour un produit cytotoxique. Les plantes résultant de cette transformation comprennent toute l'ADN en question dont le gène ne s'exprime que dans les microspores. Cependant, à ce stade, la plante étant diploïde au moment de la transformation, elle devient hétérozygote quant à sa stérilité mâle et n'est donc capable, après transformation, de donner lieu qu'à 50 % de microspores viables (les 50 % autres étant détruits suite à l'expression du transformant).

A l'étape b), la restauration ou l'induction de la fertilité perdue par la plante transformée est donc ensuite réalisée par l'inhibition de la toxicité du produit du gène transformant.

Ceci peut se faire de diverses manières, cependant, dans le cas où le produit cytotoxique en question est une subtilisine et en particulier, la subtilisine BPN' de *Bacillus amyloliquefasciens,* l'inhibition est réalisée par l'action sur la plante transformée d'une molécule insecticide de la famille des phosphofluorés (n'ayant donc *a priori* pas d'action sur les plantes). En effet, cette molécule appliquée durant l'anthèse, est susceptible de restaurer la fertilité totale des plantes hémizygotes par inhibition de la subtilisine. Elle peut par exemple être appliquée au pied de la plante et atteindre tous les tissus. Comme un insecticide, elle ne devrait avoir aucun effet sur la plante, cependant, elle jouera son plein effet au niveau des microspores, seuls organes exprimant la subtilisine.

Ensuite, à l'étape c), on procède à l'autofécondation des plantes dont la fertilité a été restaurée puis, à l'étape d), on sélectionne les plantes homozygotes quant à la stérilité mâle et par conséquent totalement stériles en l'absence de traitement c'est-à-dire d'inhibition du produit cytotoxique.

Les plantes ainsi obtenues, incapables de produire des gamètes mâles mais toujours capables de produire des gamètes femelles, c'est-à-dire des ovules, peuvent donc être croisées avec une autre lignée de plantes totalement fertiles et présentant des caractéristiques agronomiques intéressantes. Dans ce croisement, la plante homozygote quant à la stérilité mâle joue le rôle de parent femelle tandis que l'autre plante joue le rôle de parent mâle. L'hybride résultant de ce croisement est hémizygote quant à la stérilité mâle et est donc susceptible de produire 50 % de grains de pollen viables (les autres portant le transgène sont donc détruits par le produit cytotoxique). Une production de 50 % du pollen est amplement suffisante pour donner lieu à des graines présentant les qualités de chacune des lignées croisées que l'on cherchait précisément à associer.

La présente invention concerne donc également un procédé d'obtention de plantes hybrides caractérisé en ce qu'il comprend le croisement de plantes de lignée A présentant une stérilité mâle gamétophytique comme ci-dessus décrit avec des plantes de lignée B d'intérêt agronomique. Elles concernent également les graines issues des plantes hybrides ainsi obtenues.

Avantageusement, les plantes conformes à l'invention appartiennent à la famille des Brassicacées ; préférentiellement, il s'agit du colza.

Par ailleurs, il est à souligner que la région promotrice conforme à l'invention peut également être mise en oeuvre dans des stratégies d'inactivation de gène par utilisation d'éléments mobiles tels que les transposons et rétrotransposons.

En effet, ceci peut être réalisé dans le but d'isoler des plantes présentant un génotype mutant stable et d'isoler de très nombreux mutants différents et indépendants.

Il s'agit de créer une séquence chimérique constituée d'une région promotrice conforme à l'invention et de la séquence, en tout ou partie, d'un élément mobile. L'expression de cet élément mobile, réduite à la phase de développement de la microspore, devrait permettre d'induire quelques mutations dans le génome des grains de pollen de la plante transformée. Il est alors possible, dans la descendance obtenue à partir de ces grains de pollen, d'isoler des individus ne portant plus le transgène mais seulement une ou plusieurs mutations issues de phénomènes de transposition. Le principe est de provoquer, grâce à la susdite région promotrice, l'activation de la transposition de ces éléments mobiles pendant un temps très court (la microsporogénèse) dans une multitude de cellules gamétiques et supprimer à la génération suivante les plantes qui portent le transgène (à savoir la région promotrice + la séquence permettant l'activation de la transposition) de façon à ce que le cycle ne recommence pas. Il s'agit ensuite de rechercher, dans la descendance et par divers techniques, les plantes pour lesquelles les éléments mobiles ont provoqués des mutations en s'insérant dans les gènes. L'étude de ces plantes permettrait notamment de comprendre la fonction du gène muté.

Parmi les éléments mobiles susceptibles d'être ainsi utilisés, on peut citer les rétrotransposons de types Tnt1, Tto1, Tnp-2, Tos10-17, Bs1, BARE-1, Ta-1, etc... ou les transposons de type Ac/Ds, Spm, Mu, etc...

La figure 1 illustre l'alignement des séquences des deux ADNc M3 (SEQ ID N° 1) et M3.21 (SEQ ID N° 2) issues des criblages de la banque ADNc de microspore de *Brassica napus cν.* Brutor. Les codons de début (ATG) et de fin (TGA) de la séquence codante putative sont soulignées.

La figure 2 donne la séquence nucléotidique du clone BnM3.4 (SEQ ID N° 3) dont l'ADNc de M3 serait issu. L'ATG en gras (position 2085) est celui qui a la plus forte probabilité d'être l'ATG fonctionnel. L'ATG souligné en position 2112 est celui qui est présent dans la séquence de l'ADNc de M3.21. L'ATG souligné en position 1965 est le premier ATG rencontré. La séquence précédant ces ATG est considérée par conséquent comme la région promotrice du gène BnM3.4.

La figure 3 illustre l'hybridation de type Northern blot avec la sonde M3 marquée au ³²P sur des ARN totaux (10 µg par puits) extraits de différents tissus de colza. **A** : boutons de 0-2 mm (méïocytes) ; **B** : boutons de 2-3 mm (microspores uninucléées); **C** : boutons de 3-4 mm (microspores binucléées); **D** : boutons supérieurs à 4 mm (grains de pollen matures) ; **E** : sépales de colza ; **F** : pistils de colza ; **G** : boutons de colza mâle stérile ; **H** : boutons entiers de colza.

La figure 4 illustre la préparation du plasmide pJD51 de 7152 pb à partir du plasmide pAFI de 5 135 pb (plasmide d'origine : pBluescript SK- PROMEGA) et du plasmide pBnB2 de 5458 pb (plasmide d'origine pBS SK- PROMEGA).

La figure 5 illustre la préparation du plasmide pJD101 1 de 19670 pb à partir du plasmide pEC2 de 15400 pb dérivé du plasmide pDHB 321.1, (D. Bouchez, communication personnelle) et du plasmide pJD51 (cf figure 2).

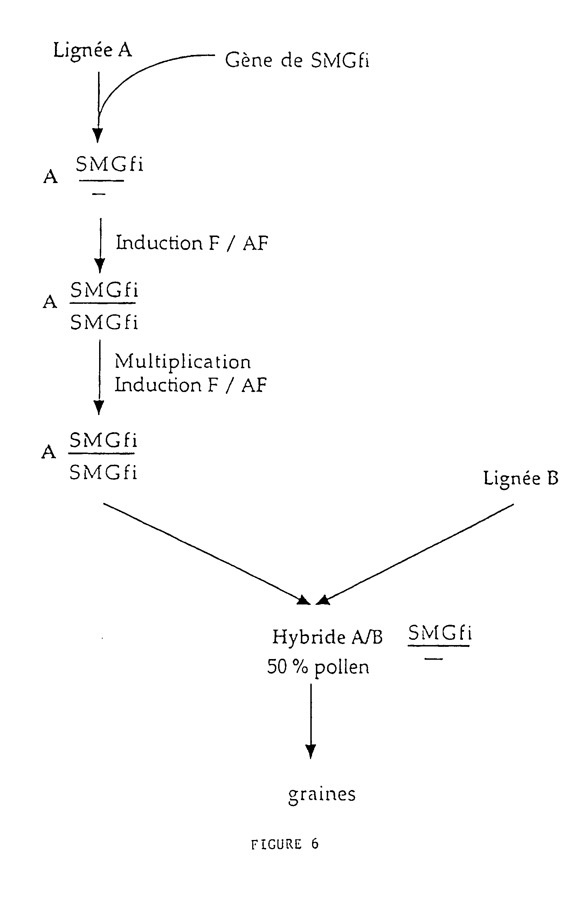

La figure 6 représente un schéma de sélection de variétés hybrides d'une plante (colza par exemple) faisant appel à un système de stérilité mâle gamétophytique avec induction de la fertilité. SMGfi : stérilité mâle gamétophytique à fertilité inductible; Induction F : induction de la fertilité; AF : autofécondation.

L'invention ne se limite pas à la seule description ci-dessus, elle sera mieux comprise à la lumière des exemples ci-après qui ne sont cependant donnés qu'à titre purement illustratif.

### EXEMPLE 1 : Mise en évidence d'un promoteur spécifique des microspores

La première étape a consisté en l'obtention de clones d'ADN complémentaires (ADNc) exprimés spécifiquement dans la microspore de colza. Pour cela, des ADNc ont été synthétisés à partir d'ARN messagers (ARNm) de microspores de colza. Parallèlement, des ADNc ont été synthétisés à partir d'ARNm de boutons floraux de colza mâles stériles. Les ADNc provenant desdits boutons floraux ont été soustraits des ADNc dérivés des ARNm exprimés dans la microspore de colza. Les molécules résultant de cette soustraction ont été utilisées lors d'une expérience d'hybridation différentielle d'une banque d'ADNc de microspore selon une technique similaire à celle présentée par Atanassov et al. (1996).

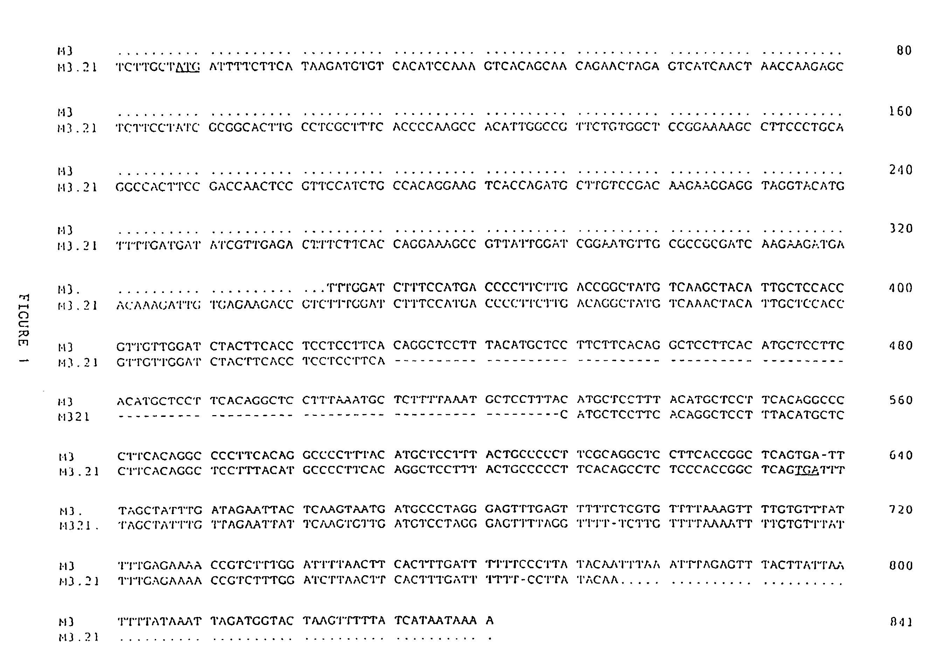

L'un de ces clones isolés, l'ADNc M3 (SEQ ID N° 1) s'est avéré être le représentant d'un ARNm spécifiquement exprimé dans la microspore de colza. Un autre ADNc, nommé M3.21 (SEQ ID N° 2) a été trouvé par criblage de la banque avec l'ADNc M3. Les séquences de ces deux ADNc présentent 89 % d'identité (figure 1), ils sont issus visiblement d'une famille de gènes très proches, exprimés spécifiquement dans la microspore.

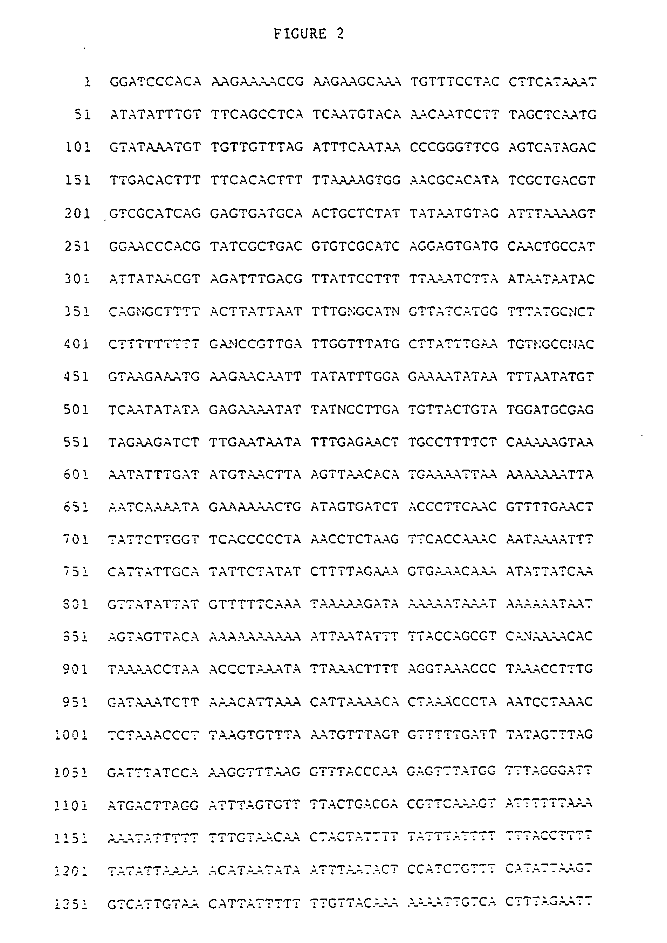

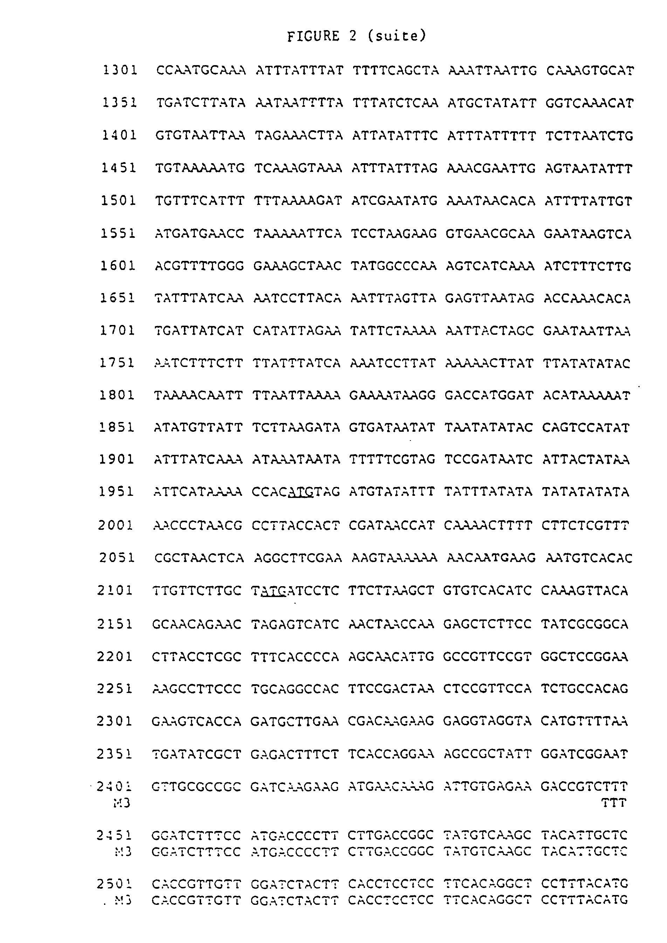

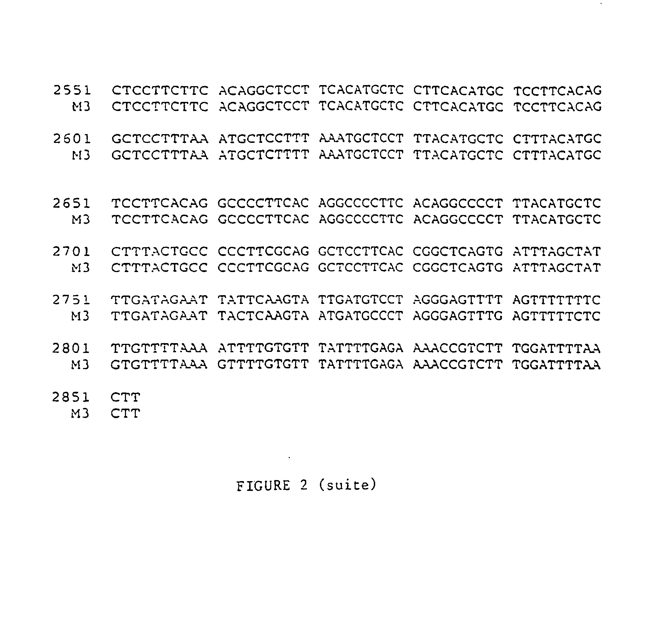

Le clone d'ADNc M3 a servi de sonde pour cribler une banque d'ADN génomique de colza commercialisée par CLONTECH Laboratories, Inc., 4030 Fabian Way, Palo Alto, CA 94303-4607, USA ; deux clones (BnM3.4 et BnM3.2) correspondants à deux gènes différents ont été isolés. L'ADNc M3 serait issu du gène BnM3.4 (SEQ ID N° 3) car celui-ci porte un orf identique à l'ADNc M3 (figure 2). Ce gène ne possède pas d'intron. Suffisamment de résultats expérimentaux laissent à penser que l'ADNc M3.21 ne serait pas issu du deuxième gène isolé (BnM3.2) qui porte bien une région correspondant à la séquence de l'ADNc M3.21, mais à un troisième gène, très proche du gène BnM3.2.

La région promotrice de ce gène est définie comme étant la séquence immédiatement en amont du codon de début de traduction (ATG).

### EXEMPLE 2: Vérification de la spécificité du promoteur du gène BnM3.4

### A/ Northern blot

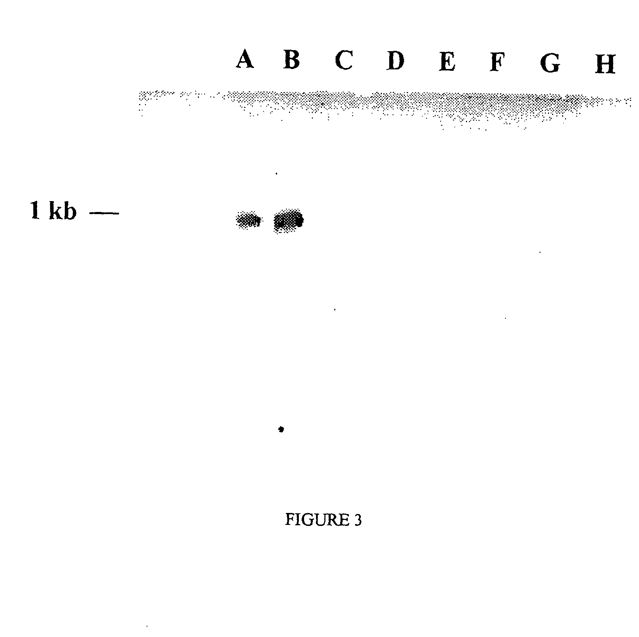

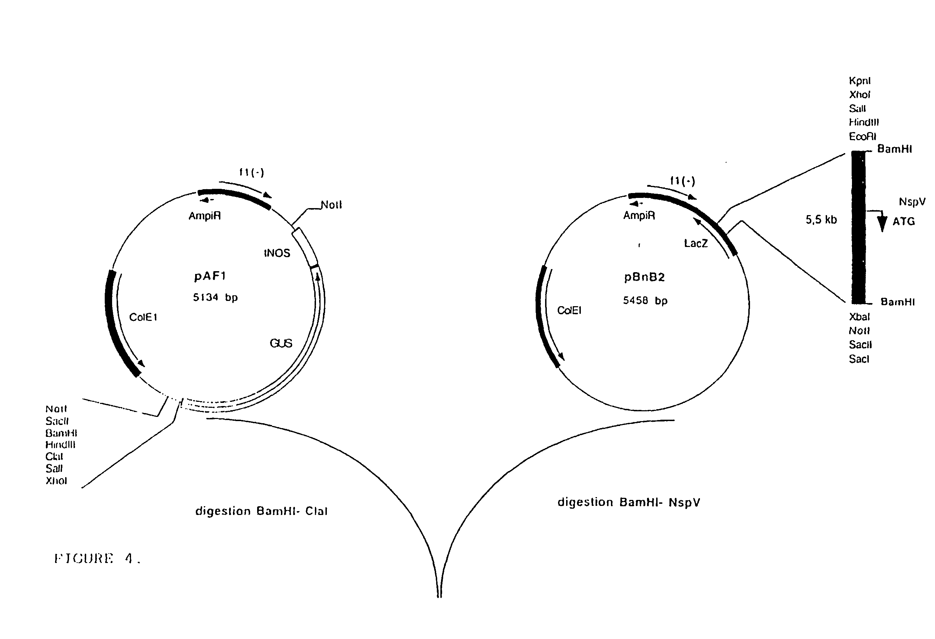

Une analyse par Northern blot a été effectuée avec 10 µg d'ARN total de sépales, de pistil, de boutons entiers, de boutons de plante mâle stérile, de méïocytes, de microspores, de grains de pollen binucléés et de grains de pollen trinucléés, hybridés avec l'ADNc M3. Une bande de 1 kb correspond au transcrit du gène BnM3.4 et aussi au transcrit M3.21, puisqu'ils ont des séquences très proches. Ces transcrits sont uniquement présents dans les deux premiers stades de la gamétogenèse mâle dont les produits sont difficiles à isoler parfaitement de façon expérimentale (figure 3).

Les protéines déduites de ces deux clones d'ADNc sont évidemment très proches et sont riches en glycine et proline. Elles ne sont strictement identiques à aucune autre protéine des banques de données mais interviennent certainement dans la constitution de la paroi.

### B/ Transformation par un gène chimérique

Différents gènes chimériques (c'est-à-dire constitués de la séquence codante d'un gène connu précédée de la région promotrice conforme à l'invention) ont été construits afin d'étudier la spécificité spatio-temporelle du promoteur de BnM3.4.

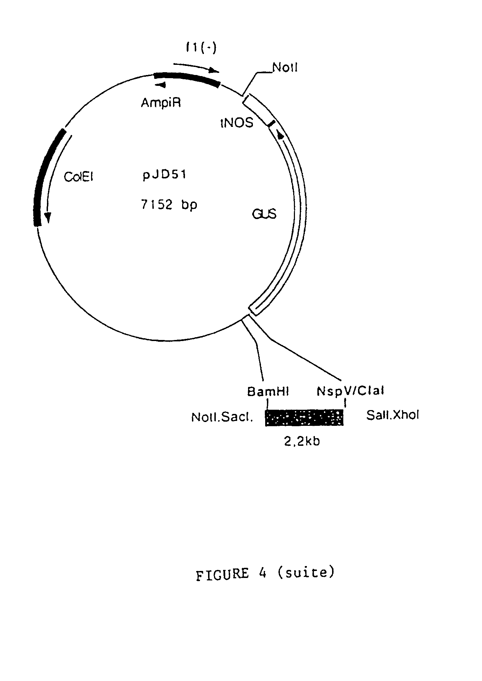

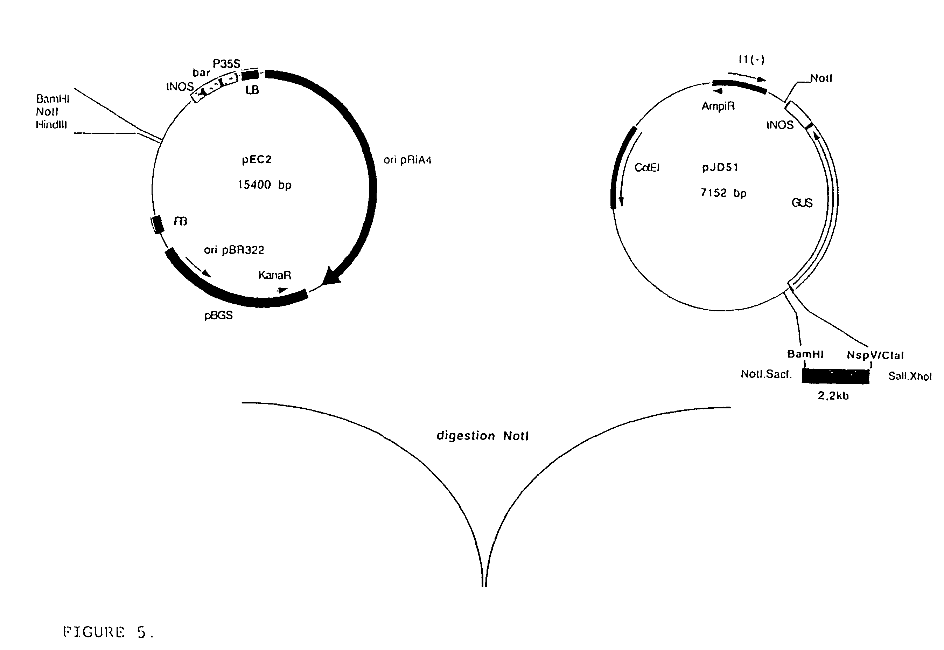

La figure 4 présente la construction d'un vecteur bactérien pJD51 associant un fragment du promoteur BnM3.4 avec la séquence codante du gène de la β-glucuronidase. Le plasmide pAF1 contenant la séquence codante de β-glucuronidase et la séquence de terminaison de transcription du gène NOS d'*Agrobacterium tumefaciens* a été digéré par les enzymes BamHI et CIaI. Le plasmide pBnB2 contient un fragment BamHI-BamHI de 6 kb issu du clone d'ADN génomique BnM3.4 et dans lequel est présent le gène BnM3.4. Un fragment correspondant à la plus grande région promotrice possible compte tenu des sites de restriction (2056 pb) a été isolé du plasmide pBnB2 par une double digestion BamHI-NspV et inséré entre les sites BamHI et CIaI (compatible avec NspV) du plasmide pAF1.

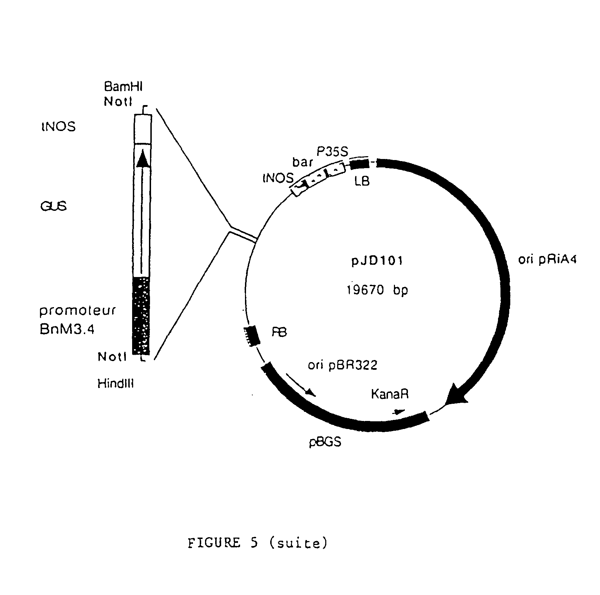

Le gène chimérique ainsi construit a été isolé par une digestion NotI du plasmide pJD51 pour être cloné dans un plasmide binaire *d'Agrobacterium tumefaciens* : pEC2 ouvert par l'enzyme NotI (figure 5).

Le plasmide binaire pJD101 contenant le gène chimérique a été introduit dans la souche C58C1 (pMP90) d*'Agrobacterium tumefasciens* (Koncz et al. 1986) par électroporation et les transformants possédant le pJD101 ont été sélectionnés sur un milieu contenant de la kanamycine. Un de ces transformants d'*Agrobacterium* a été utilisé pour transformer *Arabidopsis thaliana* (écotype Wassilevskja) par la méthode d'infiltration des hampes florales décrite par Bechtold et al. 1993. Les plantes transformées sont sélectionnées grâce à leur résistance à la phosphinothrycine, conférée par un gène de résistance inséré conjointement dans l'ADN-T.

Parmi ces plantes, certaines présentent une expression de la β-glucuronidase spécifiquement dans les microspores (mis en évidence par une coloration bleue lors de l'ajout d'un substrat spécifique de la β-glucuronidase, le X-Glu). Aucune coloration n'est présente dans les tissus adjacents de l'anthère de même que dans les tissus somatiques de la plante. Chez une plante transformée hémizygote pour le gène chimérique, la moitié des microspores produites sont bleues car elles seules contiennent le gène chimérique.

La spécificité d'expression conférée par cette séquence promotrice de 2 kb est bien restreinte, dans les limites de la sensibilité de la technique, à un seul type cellulaire et à partir du stade microspore.

### REFERENCES

Atanassov I et al. (1996) Plant Science 118, 185-194

Bechtold N. et al (1993) Comptes-Rendus de l'Académie des Sciences 316, 1194-1199

Koncz et al. 1986) Molecular General Genetics 204, 383-396

Mariani et al. Nature 347(1990) 737-741

Oldenholf M.T. et al. (1996) Plant Molecular Biology 31, 213-225

Siemens and Schieder 1996. Plant Tissue Culture and Biotechnology, 2, 66-75

## Revendications

1. Séquence nucléotidique correspondant à la séquence selon SEQ ID N° 3.

2. Séquence nucléotidique selon la revendication 1 correspondant à la séquence s'étendant du nucléotide 1 au nucléotide 2111, de préférence du nucléotide 1 au nucléotide 2084 de SEQ ID N° 3.

3. Vecteur d'expression cellulaire comprenant une séquence selon la revendication 2 placée en amont d'une séquence d'ADN codant pour un produit cytotoxique.

4. Vecteur selon la revendication 3, **caractérisé en ce que** le produit cytotoxique est une protéase et de préférence une subtilisine.

5. Cellules de plante transformées par un vecteur selon la revendication 3 ou 4.

6. Plantes présentant une stérilité mâle gamétophytique à fertilité inductible comprenant des cellules selon la revendication 5.

7. Procédé d'obtention de plantes présentant une stérilité mâle gamétophytique à fertilité inductible telles que définies dans la revendication 6, comprenant :
- l'insertion dans des plantes d'une lignée A d'un gène dont le produit d'expression est cytotoxique pour les microspores, et
- l'obtention de plantes ne produisant pas de gamètes mâles.

8. Procédé d'obtention de plantes présentant une stérilité mâle gamétophytique à fertilité inductible selon la revendication 7 comprenant les étapes de :
a) transformation de plantes d'une lignée A avec un vecteur selon la revendication 3 ou 4,
b) induction de la fertilité des plantes obtenues en a) par inhibition de la cytotoxicité du produit,
c) autofécondation des plantes fertiles obtenues en b),
d) sélection des plantes ne produisant pas de gamètes mâles issues de c),
e) multiplication des plantes obtenues en d) par reproduction des étapes b) et c).

9. Procédé d'obtention de plantes selon la revendication 7 ou 8, **caractérisé en ce que**, dans le cas où le produit cytotoxique est une subtilisine, l'induction de la fertilité consiste à appliquer à la plante une molécule insecticide de la famille des phosphofluorés.

10. Graines présentant une stérilité mâle gametophytique à fertilité induct issues des plantes hybrides obtenues par croisement de plantes de lignée A homozygote présentant une stérilité mâle gamétophytique à fertilité inductible selon la revendication 6 ou tel qu'obtenues par la mise en oeuvre du procédé selon l'une des revendications 7 à 9 avec des plantes de lignée B d'intérêt agronomique.

11. Plantes selon la revendication 6 ou obtenues par la mise en oeuvre d'un procédé selon l'une quelconque des revendication 7 à 9, **caractérisées en ce qu'**elles appartiennent à la famille des Brassicacées et de préférence **en ce qu'**il s'agit de colza.

## Claims

1. Nucleotide sequence corresponding to the sequence according to SEQ ID No. 3.

2. Nucleotide sequence according to Claim 1, corresponding to the sequence which stretches from nucleotide 1 to nucleotide 2111, preferably from nucleotide 1 to nucleotide 2084 of SEQ ID No, 3.

3. Cellular expression vector, comprising a sequence according to Claim 2, placed upstream of a DNA sequence encoding a cytotoxic product.

4. Vector according to Claim 3, **characterized in that** the cytotoxic product is a protease and preferably a subtilisin.

5. Plant cells transformed with a vector according to Claim 3 or 4.

6. Plants with gametophytic male sterility with inducible fertility, comprising cells according to Claim 5.

7. Method for producing plants with gametophytic male sterility with inducible fertility as defined in Claim 6, comprising:
- the insertion into plants of a line A of a gene whose expression product is cytotoxic for the microspores, and
- the production of plants which do not produce male gametes.

8. Method for producing plants with gametophytic male sterility with inducible fertility according to Claim 7, comprising the steps of:
a) transformation of plants of a line A with a vector according to Claim 3 or 4,
b) induction of the fertility of the plants obtained in a) by inhibition of the cytotoxicity of the product,
c) self-fertilization of the fertile plants obtained in b),
d) selection of the plants which do not produce male gametes, derived from c),
e) multiplication of the plants obtained in
d) by reproduction of steps b) and c).

9. Method for producing plants according to Claim 7 or 8, **characterized in that**, when the cytotoxic product is a subtilisin, the induction of the fertility consists in applying to the plant an insecticide molecule of the fluorophosphate family.

10. Seeds having gametophytic male sterility with inducible fertility, derived from the hybrid plants obtained by crossing plants of homozygous line A, which have gametophytic male sterility with inducible fertility, according to Claim 6, or as obtained by using the method according to one of Claims 7 to 9, with plants of line B of agronomic value.

11. Plants according to Claim 6, or obtained by using a method according to any one of Claims 7 to 9, **characterized in that** they belong to the Brassicacea family and preferably **in that** they are rape.

## Patentansprüche

1. Nukleotidsequenz, welche der Sequenz gemäß SEQ ID Nr. 3 entspricht.

2. Nukleotidsequenz nach Anspruch 1, welche der Sequenz entspricht, die sich von Nukleotid 1 bis Nukleotid 2111, vorzugsweise von Nukleotid 1 bis Nukleotid 2084 von SEQ ID Nr. 3 erstreckt.

3. Vektor für eine zelluläre Expression, welcher eine Sequenz nach Anspruch 2, welche sich stromaufwärts von einer DNA-Sequenz, welche ein zytotoxisches Produkt kodiert, befindet, umfasst.

4. Vektor nach Anspruch 3, **dadurch gekennzeichnet, dass** das zytotoxische Produkt eine Protease und vorzugsweise ein Subtilisin ist.

5. Durch einen Vektor nach Anspruch 3 oder 4 transformierte Pflanzenzellen.

6. Pflanzen, welche eine gametophytische männliche Sterilität mit induzierbarer Fertilität aufweisen, welche Zellen nach Anspruch 5 umfassen.

7. Verfahren zur Herstellung von Pflanzen, welche eine gametophytische männliche Sterilität mit induzierbarer Fertilität aufweisen, wie in Anspruch 6 definiert, umfassend:
- die Insertion eines Gens, dessen Expressionsprodukt für die Mikrosporen zytotoxisch ist, in Pflanzen einer Linie A und
- die Gewinnung von Pflanzen, die keine männlichen Gameten produzieren.

8. Verfahren zur Herstellung von Pflanzen, welche eine gametophytische männliche Sterilität mit induzierbarer Fertilität aufweisen, nach Anspruch 7, umfassend die Schritte:
a) Transformation von Pflanzen einer Linie A mit einem Vektor nach Anspruch 3 oder 4,
b) Induktion der Fertilität der in a) erhaltenen Pflanzen durch Inhibition der Zytotoxizität des Produkts,
c) Selbstbestäubung der in b) erhaltenen fertilen Pflanzen,
d) Selektion der aus c) hervorgegangenen Pflanzen, die keine männlichen Gameten produzieren,
e) Vermehrung der in d) erhaltenen Pflanzen durch Wiederholung der Schritte b) und c).

9. Verfahren zur Herstellung von Pflanzen nach Anspruch 7 oder 8, **dadurch gekennzeichnet, dass** in dem Falle, wo das zytotoxische Produkt ein Subtilisin ist, die Induktion der Fertilität darin besteht, auf die Pflanze ein insektizid wirksames Molekül aus der Familie der Phosphor und Fluor enthaltenden Verbindungen aufzubringen.

10. Saatgut, welches eine gametophytische männliche Sterilität mit induzierbarer Fertilität aufweist, welches von den hybriden Pflanzen stammt, welche durch Kreuzung von Pflanzen der homozygoten Linie A, welche eine gametophytische männliche Sterilität mit induzierbarer Fertilität aufweist, nach Anspruch 6 oder wie erhalten durch das Ausführen des Verfahrens nach einem der Ansprüche 7 bis 9, mit Pflanzen der Linie B von agronomischem Interesse erhalten werden.

11. Pflanzen nach Anspruch 6 oder erhalten durch das Ausführen eines Verfahrens nach einem der Ansprüche 7 bis 9, **dadurch gekennzeichnet, dass** sie zu der Familie der Brassicaceae gehören, und vorzugsweise, dass es sich um Colza handelt.


### LISTE DE SEQUENCES

(1) INFORMATIONS GENERALES:
   (i) DEPOSANT:
      (A) NOM: INRA (INSTITUT NATIONAL DE LA RECHERCHE AGRONOMIQUE)
      (B) RUE: 147 RUE DE L'UNIVERSITE
      (C) VILLE: PARIS
      (E) PAYS: FRANCE
      (F) CODE POSTAL: 75007
   (ii) TITRE DE L' INVENTION: Promoteur spécifique de microspores et procédé d'obtention de plantes hybrides
   (iii) NOMBRE DE SEQUENCES: 3
   (iv) FORME DECHIFFRABLE PAR ORDINATEUR:
      (A) TYPE DE SUPPORT: Floppy disk
      (B) ORDINATEUR: IBM PC compatible
      (C) SYSTEME D'EXPLOITATION: PC-DOS/MS-DOS
      (D) LOGICIEL: Patent In Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 497 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: M3
   (xi) DESCRIPTION DE LA SEQUENCE: SEQ ID NO: 1:
   TTTGGATCTT TCCATGACCC CTTCTTGACC GGCTATGTCA AGCTACATTG CTCCACCGTT    60
   GTTGGATCTA CTTCACCTCC TCCTTCACAG GCTCCTTTAC ATGCTCCTTC TTCACAGGCT   120
   CCTTCACATG CTCCTTCACA TGCTCCTTCA CAGGCTCCTT TAAATGCTCT TTTAAATGCT   180
   CCTTTACATG CTCCTTTACA TGCTCCTTCA CAGGCCCCTT CACAGGCCCC TTCACAGGCC   240
   CCTTTACATG CTCCTTTACT GCCCCCTTCG CAGGCTCCTT CACCGGCTCA GTGATTTAGC   300
   TATTTGATAG AATTACTCAA GTAATGATGC CCTAGGGAGT TTGAGTTTTT CTCGTGTTTT   360
   AAAGTTTTGT GTTTATTTTG AGAAAACCGT CTTTGGATTT TAACTTCACT TTGATTTTTT   420
   CCCTTATACA ATTTAAATTT AGAGTTTACT TATTAATTTT ATAAATTAGA TGGTACTAAG   480
   TTTTTATCAT AATAAAA                                                  497
(2) INFORMATIONS POUR LA SEQ ID NO: 2:
   (i) CARACTERISTIQUES DE LA SEQUENCE:
      (A) LONGUEUR: 674 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: M3.21
   (xi) DESCRIPTION DE LA SEQUENCE: SEQ ID NO: 2:
   TCTTGCTATG ATTTTCTTCA TAAGATGTGT CACATCCAAA GTCACAGCAA CAGAACTAGA    60
   GTCATCAACT AACCAAGAGC TCTTCCTATC GCGGCACTTG CCTCGCTTTC ACCCCAAGCC   120
   ACATTGGCCG TTCTGTGGCT CCGGAAAAGC CTTCCCTGCA GGCCACTTCC GACCAACTCC   180
   GTTCCATCTG CCACAGGAAG TCACCAGATG CTTGTCCGAC AAGAAGGAGG TAGGTACATG   240
   TTTTGATGAT ATCGTTGAGA CTTTCTTCAC CAGGAAAGCC GTTATTGGAT CGGAATGTTG   300
   CGCCGCGATC AAGAAGATGA ACAAAGATTG TGAGAAGACC GTCTTTGGAT CTTTCCATGA   360
   CCCCTTCTTG ACAGGCTATG TCAAACTACA TTGCTCCACC GTTGTTGGAT CTACTTCACC   420
   TCCTCCTTCA CATGCTCCTT CACAGGCTCC TTTACATGCT CCTTCACAGG CTCCTTTACA   480
   TGCCCCTTCA CAGGCTCCTT TACTGCCCCC TTCACAGCCT CTCCCACCGG CTCAGTGATT   540
   TTAGCTATTT GTTAGAATTA TTCAAGTGTT GATGTCCTAG GGAGTTTTAG GTTTTTCTTG   600
   TTTTAAAATT TTGTGTTTAT TTTGAGAAAA CCGTCTTTGG ATCTTAACTT CACTTTGATT   660
   TTTTCCTTAT ACAA                                                     674
(2) INFORMATIONS POUR LA SEQ ID NO: 3:
   (i) CARACTERISTIQUES DE LA SEQUENCE:
      (A) LONGUEUR: 2853 paires de bases
      (B) TYPE: nucléotide
      (C) NOMBRE DE BRINS: simple
      (D) CONFIGURATION: linéaire
   (ii) TYPE DE MOLECULE: ADNc
   (ix) CARACTERISTIQUE:
      (A) NOM/CLE: BnM3.4
   (xi) DESCRIPTION DE LA SEQUENCE: SEQ ID NO: 3:
   GGATCCCACA AAGAAAACCG AAGAAGCAAA TGTTTCCTAC CTTCATAAAT ATATATTTGT     60
   TTCAGCCTCA TCAATGTACA AACAATCCTT TAGCTCAATG GTATAAATGT TGTTGTTTAG    120
   ATTTCAATAA CCCGGGTTCG AGTCATAGAC TTGACACTTT TTCACACTTT TTAAAAGTGG    180
   AACGCACATA TCGCTGACGT GTCGCATCAG GAGTGATGCA ACTGCTCTAT TATAATGTAG    240
   ATTTAAAAGT GGAACCCACG TATCGCTGAC GTGTCGCATC AGGAGTGATG CAACTGCCAT    300
   ATTATAACGT AGATTTGACG TTATTCCTTT TTAAATCTTA ATAATAATAC CAGNGCTTTT    360
   ACTTATTAAT TTTGNGCATN GTTATCATGG TTTATGCNCT CTTTTTTTTT GANCCGTTGA    420
   TTGGTTTATG CTTATTTGAA TGTNGCCNAC GTAAGAAATG AAGAACAATT TATATTTGGA    480
   GAAAATATAA TTTAATATGT TCAATATATA GAGAAAATAT TATNCCTTGA TGTTACTGTA    540
   TGGATGCGAG TAGAAGATCT TTGAATAATA TTTGAGAACT TGCCTTTTCT CAAAAAGTAA    600
   AATATTTGAT ATGTAACTTA AGTTAACACA TGAAAATTAA AAAAAAATTA AATCAAAATA    660
   GAAAAAACTG ATAGTGATCT ACCCTTCAAC GTTTTGAACT TATTCTTGGT TCACCCCCTA    720
   AACCTCTAAG TTCACCAAAC AATAAAATTT CATTATTGCA TATTCTATAT CTTTTAGAAA    780
   GTGAAACAAA ATATTATCAA GTTATATTAT GTTTTTCAAA TAAAAAGATA AAAAATAAAT    840
   AAAAAATAAT AGTAGTTACA AAAAAAAAAA ATTAATATTT TTACCAGCGT CANAAAACAC    900
   TAAAACCTAA ACCCTAAATA TTAAACTTTT AGGTAAACCC TAAACCTTTG GATAAATCTT    960
   AAACATTAAA CATTAAAACA CTAAACCCTA AATCCTAAAC TCTAAACCCT TAAGTGTTTA   1020
   AATGTTTAGT GTTTTTGATT TATAGTTTAG GATTTATCCA AAGGTTTAAG GTTTACCCAA   1080
   GAGTTTATGG TTTAGGGATT ATGACTTAGG ATTTAGTGTT TTACTGACGA CGTTCAAAGT   1140
   ATTTTTTAAA AAATATTTTT TTTGTAACAA CTACTATTTT TATTTATTTT TTTACCTTTT   1200
   TATATTAAAA ACATAATATA ATTTAATACT CCATCTGTTT CATATTAAGT GTCATTGTAA   1260
   CATTATTTTT TTGTTACAAA AAAATTGTCA CTTTAGAATT CCAATGCAAA ATTTATTTAT   1320
   TTTTCAGCTA AAATTAATTG CAAAGTGCAT TGATCTTATA AATAATTTTA TTTATCTCAA   1380
   ATGCTATATT GGTCAAACAT GTGTAATTAA TAGAAACTTA ATTATATTTC ATTTATTTTT   1440
   TCTTAATCTG TGTAAAAATG TCAAAGTAAA ATTTATTTAG AAACGAATTG AGTAATATTT   1500
   TGTTTCATTT TTTAAAAGAT ATCGAATATG AAATAACACA ATTTTATTGT ATGATGAACC   1560
   TAAAAATTCA TCCTAAGAAG GTGAACGCAA GAATAAGTCA ACGTTTTGGG GAAAGCTAAC   1620
   TATGGCCCAA AGTCATCAAA ATCTTTCTTG TATTTATCAA AATCCTTACA AATTTAGTTA   1680
   GAGTTAATAG ACCAAACACA TGATTATCAT CATATTAGAA TATTCTAAAA AATTACTAGC   1740
   GAATAATTAA AATCTTTCTT TTATTTATCA AAATCCTTAT AAAAACTTAT TTATATATAC   1800
   TAAAACAATT TTAATTAAAA GAAAATAAGG GACCATGGAT ACATAAAAAT ATATGTTATT   1860
   TCTTAAGATA GTGATAATAT TAATATATAC CAGTCCATAT ATTTATCAAA ATAAATAATA   1920
   TTTTTCGTAG TCCGATAATC ATTACTATAA ATTCATAAAA CCACATGTAG ATGTATATTT   1980
   TATTTATATA TATATATATA AACCCTAACG CCTTACCACT CGATAACCAT CAAAACTTTT   2040
   CTTCTCGTTT CGCTAACTCA AGGCTTCGAA AAGTAAAAAA AACAATGAAG AATGTCACAC   2100
   TTGTTCTTGC TATGATCCTC TTCTTAAGCT GTGTCACATC CAAAGTTACA GCAACAGAAC   2160
   TAGAGTCATC AACTAACCAA GAGCTCTTCC TATCGCGGCA CTTACCTCGC TTTCACCCCA   2220
   AGCAACATTG GCCGTTCCGT GGCTCCGGAA AAGCCTTCCC TGCAGGCCAC TTCCGACTAA   2280
   CTCCGTTCCA TCTGCCACAG GAAGTCACCA GATGCTTGAA CGACAAGAAG GAGGTAGGTA   2340
   CATGTTTTAA TGATATCGCT GAGACTTTCT TCACCAGGAA AGCCGCTATT GGATCGGAAT   2400
   GTTGCGCCGC GATCAAGAAG ATGAACAAAG ATTGTGAGAA GACCGTCTTT GGATCTTTCC   2460
   ATGACCCCTT CTTGACCGGC TATGTCAAGC TACATTGCTC CACCGTTGTT GGATCTACTT   2520
   CACCTCCTCC TTCACAGGCT CCTTTACATG CTCCTTCTTC ACAGGCTCCT TCACATGCTC   2580
   CTTCACATGC TCCTTCACAG GCTCCTTTAA ATGCTCCTTT AAATGCTCCT TTACATGCTC   2640
   CTTTACATGC TCCTTCACAG GCCCCTTCAC AGGCCCCTTC ACAGGCCCCT TTACATGCTC   2700
   CTTTACTGCC CCCTTCGCAG GCTCCTTCAC CGGCTCAGTG ATTTAGCTAT TTGATAGAAT   2760
   TATTCAAGTA TTGATGTCCT AGGGAGTTTT AGTTTTTTTC TTGTTTTAAA ATTTTGTGTT   2820
   TATTTTGAGA AAACCGTCTT TGGATTTTAA CTT                                2853